# EUROPEAN PATENT APPLICATION

(11) **EP 3 719 809 A1**
(43) Date of publication of application: **07.10.2020**
(21) Application number: 19167425.8
(22) Date of filing: 04.04.2019
(51) Int. Cl.: G16H 50/20

(54) **METHOD AND SYSTEM FOR MEDICAL DIAGNOSIS ASSISTANCE**

(71) Applicant: Koninklijke Philips N.V., 5656 AG Eindhoven (NL)
(72) Inventor: TALGORN, Elise Claude Valentine, 5656 AE Eindhoven (NL); IN 'T GROEN, Robertus Leonardus Maria, 5656 AE Eindhoven (NL); LAUTE, Niels, 5656 AE Eindhoven (NL); GEURTS, Lucas Jacobus Franciscus, 5656 AE Eindhoven (NL)
(74) Representative: de Haan, Poul Erik

(57) **Abstract**

Some embodiments are directed to a method (400) for medical diagnosis assistance. The method may include recording (410) a conversation of a patient using an electronic recording system, and providing (440) the first and second topic to a display to be presented to a clinician. The conversation may be a spoken conversation which is analyzed using speech recognition.

## Description

### FIELD OF THE INVENTION

The invention relates to method a computer-implemented method for medical diagnosis assistance, a computer readable medium, an electronic system for medical diagnosis assistance, and an electronic device for medical diagnosis assistance.

### BACKGROUND OF THE INVENTION

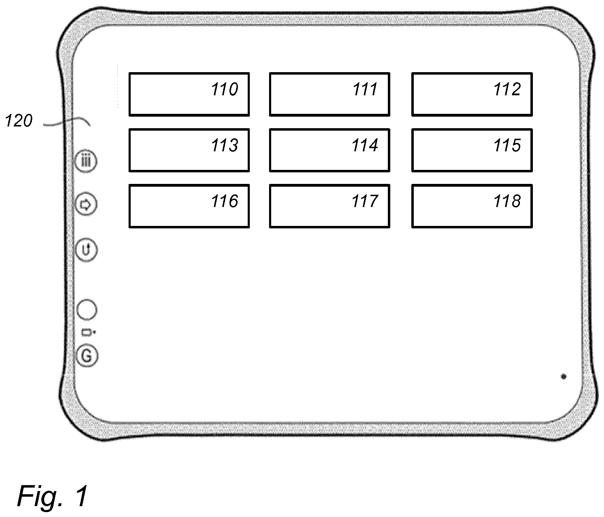

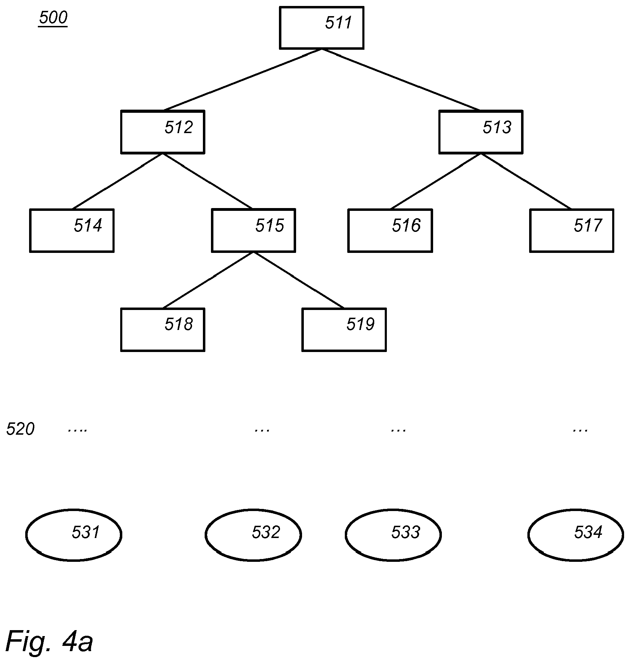

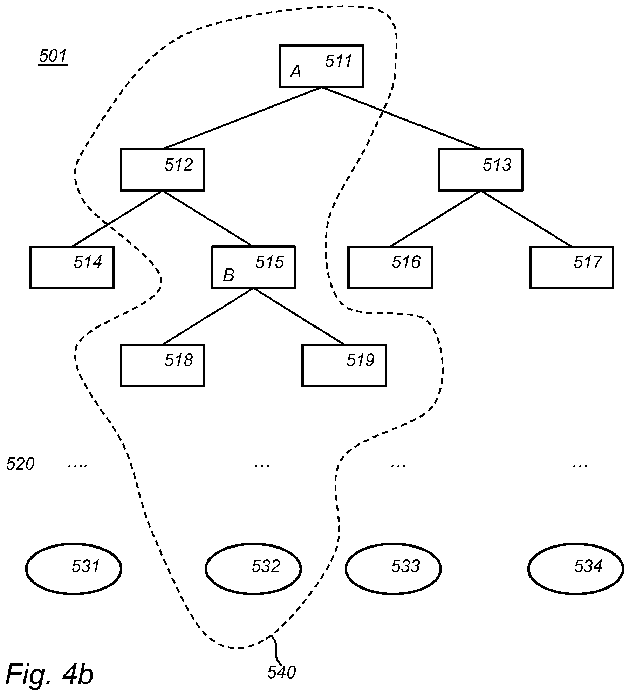

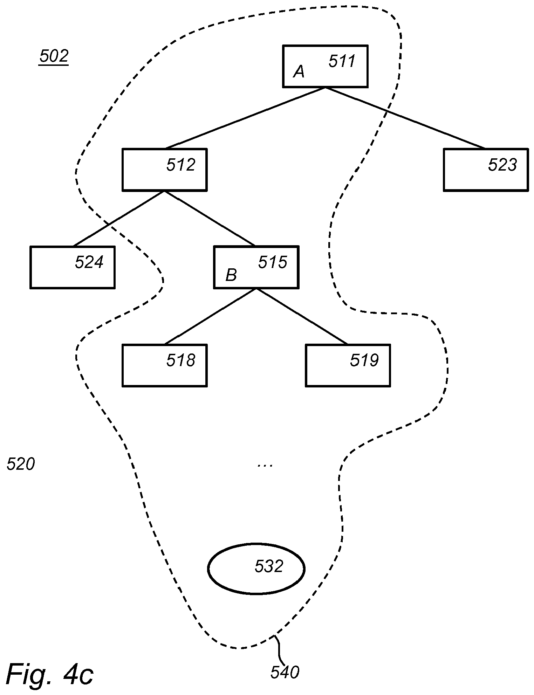

Granted EP patent application EP3281013B1 discloses a device for use in a diagnostic method. The known device assists in the assessment of the prognosis and treatment of a patient. The device comprises a tablet equipped with a computer program. Figure 1 shows the tablet according to the known system. Figure 1 in the current application has been adapted from figure 4 in EP3281013B1.

The computer program on the tablet is configured to record a selection of clinical signs and/or symptoms felt by a patient. The patient can choose from multiple signs or symptoms recorded in the computer program, which can be felt by patients suffering from any of a number of diseases. Based on the selection of the patient, the tablet may determine and indicate a biological analysis test that is to be performed to further diagnosis.

Figure 1 shows the known tablet 120 as it shows the patient a number of symptoms:

| | | | |
|---|---|---|---|
| 110 | Fever | 115 | Penile discharge |
| 111 | Stomach ache | 116 | Cough |
| 112 | Vomiting | 117 | Chest pain |
| 113 | Diarrhea | 118 | Sore throat |
| 114 | Burning when urinating | | |

For example, if options 114 and 115 are chosen, the tablet will advise the patient to take a diagnostic test for sexually transmitted diseases.

Existing diagnostic tools such as the known system have been found to be lacking. It turns out that self-reporting of symptoms in a formal setting such as presented by tablet 100 leads to underreporting of symptoms. For example, people may forget to report a symptom, may not be aware of the relevance, but above all may be too self-conscious to report certain symptoms, especially such symptoms as are related to sexually transmitted diseases, eating disorders, mental issue, and the like.

There is therefore a need for an improved device for assistance in diagnostics.

### SUMMARY OF THE INVENTION

A computer-implemented method for medical diagnosis assistance is provided, which comprises recording one or more conversations of a patient using an electronic recording system. A plurality of patient-specific topics are identified in the recorded conversations. A first topic in the plurality of patient-specific topics is classified as a medical symptom, and a second topic is determined in the plurality of patient-specific topics that is correlated with the first topic in the recorded conversations of the patient. In response to a trigger for the first topic, information associated with the second topic is presented to a clinician. For example, a method may have three phases: a recording phase in which conversations of a patient are recorded, an analysis phase in which topics and correlations are found, and a retrieval phase in which a correlated second topic is retrieved, triggered by a first topic. The phases typically occur separated in time, though they may partially overlap. In particular, the first two phases may be performed in parallel.

It was found that patients, for various reasons, may sometimes neglect to mention important information during an assessment by a clinician, e.g., during an anamnesis. For example, a patient may fail to see the relevance of the information or may be reluctant to discuss the information, e.g., if the information is sensitive, e.g., embarrassing. In the example in the background, for instance, a user may be hesitant to reveal some of the sensitive problems that are associated with his medical conditions, say that are associated with a burning feeling during urinating. Nevertheless, such additional information may very well be important to arrive at a correct diagnosis, or to order the appropriate further diagnostic testing. The additional information may have been discussed by the patient in the past, say with a friend, but traditionally a doctor cannot obtain such information. According to an embodiment, topics are identified in recorded information of a patient. Topics that are correlated with each other, e.g., because they have occurred together close in time, may be determined. For example, the method may comprise executing a speech recognition algorithm so that topics may be identified from a spoken conversation. For example, if a patient mentions the medical symptom 'burning feeling during urinating', then this may be identified. The system may determine that additional symptoms, e.g., blood in urine, pregnancy, have been mentioned in relation to the burning feeling, or non-medical symptoms, e.g., activities that may have resulted in a sexually transmitted infection (STI).

There are various ways in which the trigger and information can occur. For example, the trigger may happen because a doctor may type in the first topic or because a speech recognition algorithm determines that the first topic is mentioned during an anamnesis. For example, the first topic may be identified on a form that is digitally filled in by a user. For example, the latter two examples may use similar software as used to analyze the recorded conversations. There are various ways in which information on the second topic may be provided. For example, the second topic may be displayed on a display. For example, in response to the mention of the triggering topic 'burning feeling during urinating', the display may show 'penile discharge' as an associated second topic, etc. Instead of directly displaying the second topic, instead related information may be shown. For example, a medical decision tree may be shown to the clinician. The items in the tree that relate to the first and/or second topic may be highlighted. The medical decision tree may even be partly pre-selected based on the first and/or second topic. For example, a part of the tree that appears to be not relevant given the first and second topic may be collapsed. A collapsed part of the tree may be opened by the clinician if wanted, but in the collapsed state requires less space on the display screen and less attention, e.g., cognitive resources, of the clinician. This means that attention is guided towards the relevant part of the medical decision tree quicker. Displaying related information instead of directly displaying the second topic has the added benefit of reducing privacy concerns.

When multiple second topics correlate with a first topic, they may all be shown to the clinician. Interestingly, the correlating second topics may be rated and shown according to the rating, e.g., in an order determined by the rating. Interestingly, the rating may indicate how easily the patient appears to discuss a particular topic, e.g., the rating may indicate an inhibition towards a topic or a lack thereof. A clinician may use this information to start his or her discussion with the patient by starting with second topics that are easier to discuss for this particular patient. Note that this rating may be different for different patients. For example, two patients may both complain of headaches, and both may show correlations with work related stress and alcohol use, yet for one patient discussing alcohol use is more sensitive and for the other discussing work-stress is more sensitive. By organizing the anamnesis accordingly a more efficient and accurate result is achieved. In an embodiment, a patient may grant different levels of access for different topics.

In an aspect of the invention, an electronic system for medical diagnosis assistance is provided. Using the known system, a doctor simply could not gain access to the information that is provided by the technical means according to an embodiment.

An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

Another aspect of the invention provides a method of making the computer program available for downloading. This aspect is used when the computer program is uploaded into, e.g., Apple's App Store, Google's Play Store, or Microsoft's Windows Store, and when the computer program is available for downloading from such a store.

### BRIEF DESCRIPTION OF THE DRAWINGS

Further details, aspects, and embodiments of the invention will be described, by way of example only, with reference to the drawings. Elements in the figures are illustrated for simplicity and clarity and have not necessarily been drawn to scale. In the Figures, elements which correspond to elements already described may have the same reference numerals. In the drawings,
Figure 1 schematically shows an example of tablet configured to allow a patient to enter a number of symptoms,
Figure 2a schematically shows an example of an embodiment of an electronic system for medical diagnosis assistance,
Figure 2b schematically shows an example of an embodiment of a device for medical diagnosis assistance,
Figure 3a schematically shows an example of an embodiment of a conversation,
Figure 3b schematically shows an example of an embodiment of speech recognition data,
Figure 3c schematically shows an example of an embodiment of classified speech recognition data,
Figure 3d schematically shows an example of an embodiment of a topic tree, Figure 4a schematically shows an example of an embodiment of a medical decision tree,
Figure 4b schematically shows an example of an embodiment of a medical decision tree,
Figure 4c schematically shows an example of an embodiment of a medical decision tree,
Figure 5 schematically shows an example of an embodiment of a computer-implemented method for medical diagnosis assistance,
Figure 6a schematically shows a computer readable medium having a writable part comprising a computer program according to an embodiment,
Figure 6b schematically shows a representation of a processor system according to an embodiment.

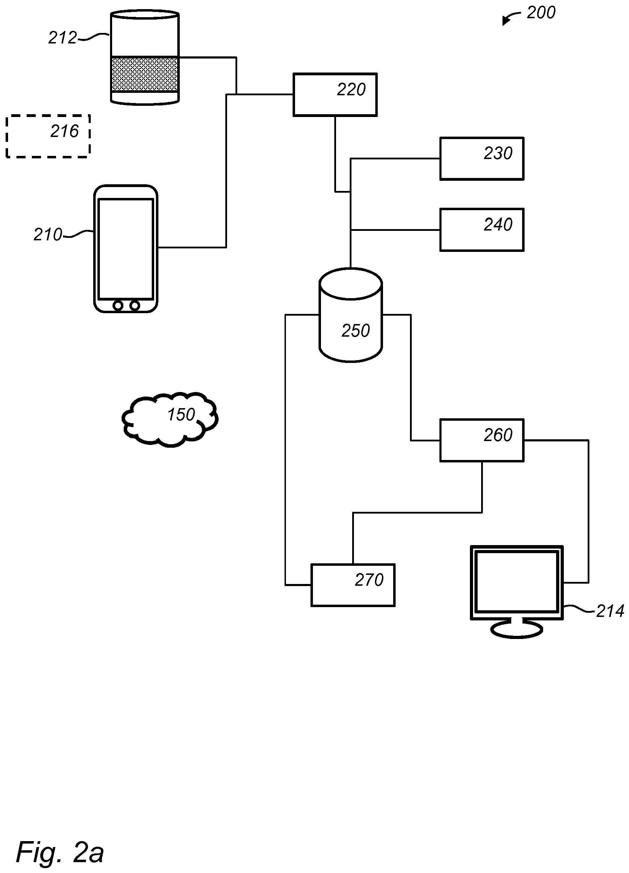

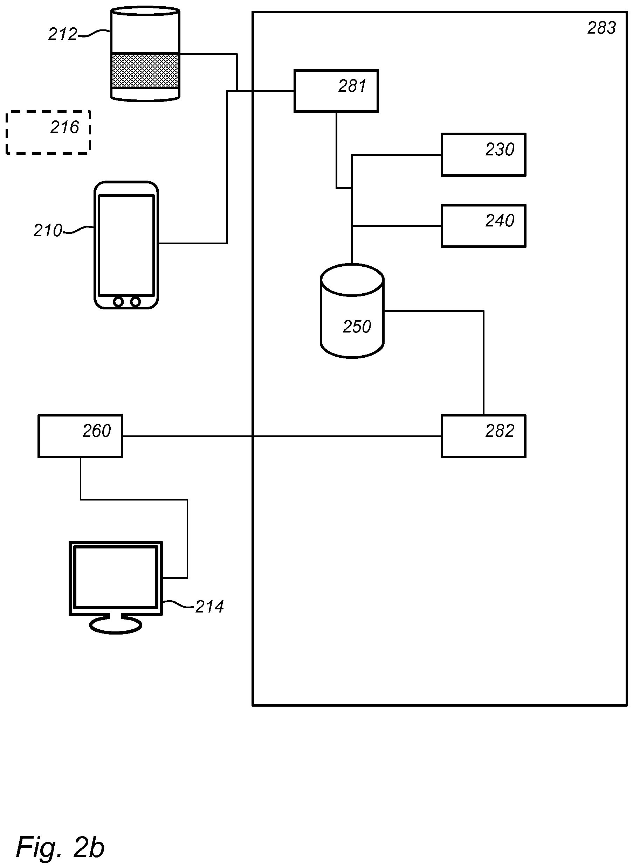

### List of Reference Numerals in figures 2, 3a-3d, 6a, 6b:

- 150: a computer network
- 200: an electronic system
- 210: a smart phone
- 212: a smart speaker
- 214: a display
- 216: recording software
- 220: a recording unit
- 230: a speech recognition unit
- 240: a topic classifier
- 250: a patient database
- 260: a clinical system
- 270: a speech recognition unit
- 281: a recording interface
- 282: a providing interface
- 283: a device for medical diagnosis assistance
- 300: a recorded conversation
- 310: speech recognition data
- 311-313: a speech recognition data item
- 320: classified speech recognition data
- 330: a topic tree
- 321-324: a topic
- 332, 333, 334: a correlation
- 500-502: a medical decision tree
- 511-519: a medical decision point
- 520: further medical decision points
- 523, 524: a collapsed sub-tree
- 531-534: a medical diagnosis
- 540: a selection

- 1000: a computer readable medium
- 1010: a writable part
- 1020: a computer program
- 1100: a device
- 1110: a system bus
- 1120: a processor
- 1130: a memory
- 1140: a user interface
- 1150: a communication interface
- 1160: a storage
- 1161: an operating system
- 1162, 1163, 1164: instructions

### DETAILED DESCRIPTION OF EMBODIMENTS

While this invention is susceptible of embodiment in many different forms, there are shown in the drawings and will herein be described in detail one or more specific embodiments, with the understanding that the present disclosure is to be considered as exemplary of the principles of the invention and not intended to limit the invention to the specific embodiments shown and described.

In the following, for the sake of understanding, elements of embodiments are described in operation. However, it will be apparent that the respective elements are arranged to perform the functions being described as performed by them. Corresponding methods may comprise performing such functions. Further, the invention is not limited to the embodiments, and the invention lies in each and every novel feature or combination of features described herein or recited in mutually different dependent claims.

Clinicians, e.g., physicians, doctors, GP, care professional, etc., have to deal with increasing workload, and shortening of standard durations for reimbursed consults. However, it requires time to gain a full understanding of the patient symptoms, concerns and related events. Especially, for patients, certain medical situations or symptoms can be awkward or embarrassing to discuss, for example, when it concerns psychological, behavioral or sexual diseases. Missing important information, e.g., due to time constraints, may lead to misdiagnosis.

Embodiments facilitate the anamnesis and/or diagnosis that a doctor performs at the beginning of a consult to understand the patient symptoms, concerns and related events, in order to get faster, more accurate answers and to avoid missing important information.

A consult performed by a clinician generally starts with the anamnesis. The clinician may ask the patient to estimate his general physical and mental state, to understand if there are specific complaints or doubts that bring the patient to the consult. For patients, certain medical situations or symptoms can be awkward or embarrassing to discuss, for example when it concerns psychological, behavioral or sexual diseases. In these cases, the patient might be reluctant to formulate his complaint or question, to factually describe the symptom or the scenario of what happened. Besides this, the patient might also feel uncomfortable when others are part of the conversation as well, e.g., a nurse, partner or parents, or there could be a cultural reason that the patient does not feel confident enough to describe the symptoms. Patients might feel insecure when describing a symptom in a non-medical, simple or 'banal' way. Finally, they can be unaware of the importance of certain symptoms or events and might neglect to mention them.

For the clinician this might mean spending a considerable amount of time questioning to understand what brings the patient to his/her office. Without probing for a true and complete view of the complaints, the clinician potentially misses complaints that are not formulated, leading to misdiagnosis.

Often patients, before going to the doctor, will search for information by asking trusted relatives or friends, or by online searches. When asking relatives or friends, people will generally start with a high-level question around the complaint or the worry they have, and if their interlocutor recognizes the situation, they may go more into specifics, e.g., into symptoms, and share more details. Conversations of a patient before seeing a clinician do not have to be limited to spoken conversation, e.g., the conversation may be typed, e.g., using a chat program. Furthermore, the conversation does not need to be with another human, but may instead be with a virtual chatbot. A chatbot may be configured for written or spoken conversation. An example of a chatbot configured for medical conversations is WoeBot, see, e.g., "The chatbot therapist will see you now" in Wired of 06.07.2017.

**Figure 2a** schematically shows an example of an embodiment of an electronic system 200 for medical diagnosis assistance. For example, system 200 may be used during anamnesis of a clinician, e.g., a doctor. For example, an embodiment of a method of medical diagnosis assistance may be employed by system 200. Figure 2a shows various elements that may be present in a system 200, including: a recording unit 220, a speech recognition unit 230, a topic classifier 240, a patient database 250, a clinical system 260, and a speech recognition unit 270. Note that not all of these parts are required. For example, if only written conversations are used, then the speech recognition unit 230 may be omitted; if no patient database is used, then patient database 250 may be omitted, etc.

The execution of the system 200 may be implemented in a processor system, e.g., on or more processor circuits. Figure 2a shows functional units that may be functional units of a processor circuit. For example, figure 2a may be used as a blueprint of a possible functional organization of one or more processor circuits. The processor system is not shown separate from the units in figure 2a. For example, the functional units shown in figure 2a may be wholly or partially implemented in computer instructions that are stored at a device, e.g., in an electronic memory of the device, and are executable by a microprocessor of device 100. In hybrid embodiments, functional units are implemented partially in hardware, e.g., as coprocessors, e.g., speech recognition coprocessors, and partially in software stored and executed on the device.

The various parts of system 200 need not be implemented on the same device. For example, in an embodiment, a first device, e.g., a mobile computing device, may comprise recording unit 220. A second device may comprise speech recognition unit 230, a topic classifier 240, a patient database 250, a clinical system 260 and a speech recognition unit 270. For example, in an embodiment, a first device, e.g., a mobile computing device, may comprise recording unit 220 and speech recognition unit 230. A second device may comprise topic classifier 240. A third device may comprise patient database 250, a fourth device may comprise clinical system 260. Examples of a mobile computing device include a smart phone, a tablet, and the like.

Many other ways of dividing system 200 over devices are possible and can be devised by a skilled person on the basis of the information contained herein.

The various devices and units of system 200 may communicate with each other over a computer network 150. The computer network may be an internet, an intranet, a LAN, a WLAN, etc. Computer network 150 may be the Internet. The computer network may be wholly or partly wired, and/or wholly or partly wireless. For example, the computer network may comprise Ethernet connections. For example, the computer network may comprise wireless connections, such as Wi-Fi, ZigBee, and the like. The devices comprise a connection interface which is arranged to communicate with other devices of system 200 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna.

For example, recording unit 220 may send one or more recorded conversations to speech recognition unit 230. This may be done over an internal communication, e.g., a bus, but if recording unit 220 and speech recognition unit 230 are not part of the same device, then this may be done over computer network 150.

For example, one or more devices in system 200 may comprise communication interfaces respectively. Computer network 150 may comprise additional elements, e.g., a router, a hub, etc. Interfaces of system 200 may be used to send and/or receive recorded conversations, topics, speech recognition data, and the like.

Recording unit 220 may be configured to record a conversation of a patient using an electronic recording system. For example, recording unit 220 may comprise or cooperate with a smart phone 210 and/or a smart speaker 212. For example, the recording system comprises a software application installed on a mobile computing device and/or a smart speaker. For example, an app may be installed on smart phone 210 and/or a smart speaker 212 to record a conversation. For example, when a user makes an appointment with the clinician, he may be offered the services of system 200. If the user consents to the recording of conversations, he may install corresponding recording software 216 on one or more of his devices, e.g., on device 212 or 210.

The conversation may be a written conversation. For example, recording software 216 may be configured for written conversation, e.g., chatting, with another real person, or with a chatbot, e.g., through a communicator app. The conversation may be a spoken conversation. For example, recording software 216 may be configured for spoken conversation, e.g., talking, with another real person, or with a chatbot configured for speech. The chatbot may be connected to software 216 via computer network 150. For example, the chatbot may be run at a remote location. The patient may be invited to discuss his medical concerns with the other person and/or with the chatbot. Recording system 220, e.g., comprising software 216, may record multiple conversations. In an embodiment, a user may converse with the chatbot, or even with another person, through existing software on his devices, e.g., device 212 or 210. For example, a user may connect to a chatbot, e.g., which may be operated by the clinician, e.g., by a hospital, e.g., through a web-browser, etc.

System 200 may comprise a speech recognition unit 230. Speech recognition unit 230 is configured to execute a speech recognition algorithm. The speech recognition algorithm is configured to generate speech recognition data. For example, speech recognition unit 230 may comprise an automatic speech recognition (ASR) toolkit, e.g., the Kaldi ASR, e.g., version 5.5.0. Recording device 220 may associate recorded conversations with a particular user, e.g., through a patient identifier. Speech recognizer 230 may be configured to recognize the sound of a particular speaker, e.g., to recognize the speech of the patient. Speech recognizer 230 may associate the analyzed data with the appropriate speaker.

Recording system 220 may be configured to record a search of the patient in an electronic information system. For example, a patient may search for medical symptoms, etc., and/or other topics, in an internet search engine. The search queries may be stored as are the conversations, e.g., a series of consecutive utterances. Utterances of the series may be associated with a topic, and topics may be correlated.

The recording system may record discussions of the patient with relatives, e.g., in a call, from a smart speaker like Alexa, in a chat app, etc., or with an artificial chatbot. The recording system may also be implemented as a web-page, e.g., a web-page offered by a hospital, at the web-page a user can talk or exchange written messages with a chatbot, and/or may search, e.g., may search a medial repository, or may search the Internet, or may talk to a person, e.g., a medical professional, etc. The patient may give consent to the recording apps and the search engine to record searches or discussions. For example, the consent may be given when a health app is installed, or a particular health web-page is consulted.

System 200 may comprise a topic classifier 240. Topic classifier 240 is configured to identify a plurality of topics in the recorded conversation. For example, topic classifier 240 may be a dialogue topic tracker. For example, topic classifier 240 may be configured for sequentially labelling the topic state at each time step in a given dialogue sequence. Topic classifier 240 may comprise a topic database comprising multiple topics. For example, the database may associate certain words or phrases with a topic. For example, the word sleeplessness may be associated with the topic insomnia. For example, the word office may be associated with the topic work. Alternatively, or in addition classifier 240 may comprise a machine learning component that has been trained to recognize topics in the recorded conversation. For example, an artificial neural network may be trained for dialogue topic tracking. The machine learning component may operate on speech recognition data, but may also directly operate on the recorded conversation, e.g., the raw conversation data as recorded, e.g., a recorded speech signal.

It was an insight to combine a phrase-based topic classifier with a neural network based topic classifier. The combination of a phrase-based classifier and a neural network has the advantage that very high reliability is achieved on stock phrases. For example, a user searching for 'idiopathic insomnia' is almost certainly concerned with the topic of insomnia. This can be identified with high reliability using a database with phrases associated to topics. In this case, one of the phrases may be 'idiopathic insomnia' and the topic 'insomnia' or even 'idiopathic insomnia', etc. On the other hand, the machine learning component, e.g., the neural network, performs better on free format descriptions. For example, mapping a sentence like 'Lately my nights have been kind of restless' is hard to map to insomnia using fixed phrases, but here a neural network will perform better. Topics may be encoded by a topic identifier, e.g., a numeral. The identifier may have a primary part for the main topic, and a secondary part for a subdivision. For example insomnia may be divided into: Acute insomnia, Chronic insomnia, Comorbid insomnia, Onset insomnia, Maintenance insomnia. This has the advantage that both correlation may be made with the general topic insomnia, which may be stronger since more utterances may be about insomnia, but at the same time correlation with specific types of insomnia are also possible.

The neural network may be a deep learning network. In an embodiment, the neural network comprises multiple convolutional layers followed by a max pooling layer.

For example, in an embodiment, a text-based topic search may use the speech recognition data, e.g., to search for predefined phrases, whereas the neural network may operate directly on the speech signal. The two types of classification may be combined, e.g., as a set-union. In an alternative, the neural network reports a reliability, so that the neural network is preferred only if its reliability is above a threshold.

Topic classifier 240 is configured to determine a correlation between topics found in the recorded conversations. Note, that this may use multiple, even many, conversations. For example, consider a first user who uses phrases classified with a first topic, e.g., insomnia, and often uses phrases classified with a second topic nearby, e.g., nearby in time. For example, the second topic may be 'alcohol'. In that case, topic classifier 240 may determine a correlation between insomnia and alcohol, for this user. A second user, unrelated to the first user, may also talk about the topic insomnia but uses the topic 'work' nearby. In this case a correlation between insomnia and work may be determined by topic classifier 240. Thus for the first user, its plurality of patient-specific topics may include insomnia and alcohol, whereas for the second user, its plurality of patient-specific topics may include insomnia and work.

For example, the plurality of patient-specific topics for the first user may be, e.g., {insomnia, alcohol, drugs}, and the plurality of patient-specific topics for the second user may be, e.g., {insomnia, work, drugs}. Even though both pluralities in this example include the topics insomnia and drugs, it may be that a correlations is found between first topic insomnia and second topic drugs in the one or more recorded conversations of the first user, but not in the one or more recorded conversations of the second user. Even if correlations are found for both users, then they may be of unequal strength. For example, for the second user the correlation may be below a noticeability threshold, e.g., so that it is not shown.

A correlation between two topics may be found in the one or more recorded conversations of a patient, by determining how often a second topic occurs near a first topic. For example, a time limit may be chosen, say 5 minutes. Accordingly, Classifier 240 may determine that the topic alcohol was used within 5 minutes of the topic insomnia, say, 3 times, whereas a topic shopping was used close to insomnia only once. Instead of a fixed time-limit, classifier may weigh topics. For example, if alcohol is used near insomnia within a short duration, say 1 minute, but shopping is used within a longer duration, say 5 minutes, then alcohol may be given a higher weight. The advantage is that a topic that is used shortly after a medical topic needs only a few uses to form a strong correlation, yet the system can still from a correlation for uses that take longer, provided they occur with a higher frequency.

For example, a correlation may be a link, an association. The correlation may be a statistical correlation. For example, finding a correlation may include establishing that using a particular first topic predicts the use of a second topic. A stronger correlation may be found, if classifier 240 establishes that not-using the first topic predicts not-using the second topic. Such stronger correlation may be established by a counting algorithms as suggested above, but may also be established by computing a statistical correlation measure. Note that it is not necessary that only speech of the patient is taken into account. For example, a correlation may also be found between a topic used by the patient and a topic used by a second speaker in the same conversation. For example, a patient may talk about sleeplessness, whereas the second speaker brings up work. The system may be configured to ignore such correlations, or to take them into account, though possibly with a lower weight. Thus even if a patient does not use a topic, but the person which whom he/she speaks does use the topic, then this may cause a correlation.

Classifier 240 thus establishes a correlation between a first topic and a second topic. Particularly advantageous is the finding of correlations between medical topics, e.g., symptoms, and other topics, e.g., other medical topics, e.g., linking headaches to insomnia, or behavioral topics, e.g., linking headaches to alcohol use or to a work situation.

Interestingly, classifier 240 may establish multiple correlations between topics in the patient-specific plurality of topics. For example, insomnia may be correlated to headaches and to alcohol. For example, a correlation threshold may be defined, and all topics with a correlation above the threshold may be identified. Finding only correlated topics for medical topics significantly reduces the required resources. Consider, if *n* topics can be identified by classifier 240, of which *k* topics are medical topics, e.g., as defined on a list of medical topics. The number of potential correlation between any two topics is 1/2*n*(*n* - 1), but the number of potential correlations with a medical topic is *kn.* Assuming *k* < 1/2 (*n* - 1), then the latter number is smaller. In practice, *k* << 1/2(*n-*1), thus providing a significant performance benefit.

Note that topics and correlations may be determined across multiple modalities. For example, a user may search for sleeplessness, followed by a search for bullying at work. Later, the same user may talk to a relative about insomnia followed by a remark about stress in the office. Both instances may count towards finding a correlation between insomnia and work. Evidence for a relationship between two topics can be found in written chats with a person or with a chatbot, in spoken chats with a person or with a chatbot, in searches, and the like.

System 200 may comprise a patient database 250. Patient database 250 is not necessary but makes scaling the system easier. Through patient database 250 the system may be used simultaneously for multiple patients. For example, the multiple patients install software 216, e.g., on their phones, but re-use other parts of system 200, e.g., speech recognizer 230, topic classifier 240, and so on. For example, a patient may be assigned a patient identifier. Information regarding the patient may be stored in database 250 associated with the patient identifier. For example, the patient identifier may be stored in recording system 220. Recording system 220 may store a conversation in database 250 associated with the patient identifier. Recording system 220 may have the conversation analyzed, e.g., speech processed by unit 230 before storing, and/or analyzed for topics, before storing. Storing speech recognition data and/or topics instead of the conversation saves considerably on storage requirements, and also reduces privacy risks. For example, analysis may be done on a different device than storage in database 250. For example, finding topics and finding correlations may be distributed over two different devices. The latter device does not need access to the conversations themselves.

For example, topics may be stored in database 250, e.g., associated with a patient identifier. For example, correlations between topics may be stored in database 250, e.g., associated with a patient identifier.

System 200 may comprise a clinical system 260 and a display 214. Clinical system 260 may be configured so that a clinician can access database 250. For example, a doctor may use a clinical system 260 to search for information regarding a particular patient. For example, the clinician may query clinical system 260 for correlated topics that relate to a topic that is currently discussed, e.g., during an anamnesis. The resulting information may be presented on a display 214. Alternative presentation is possible, e.g., a print-out, a smart speaker, etc.

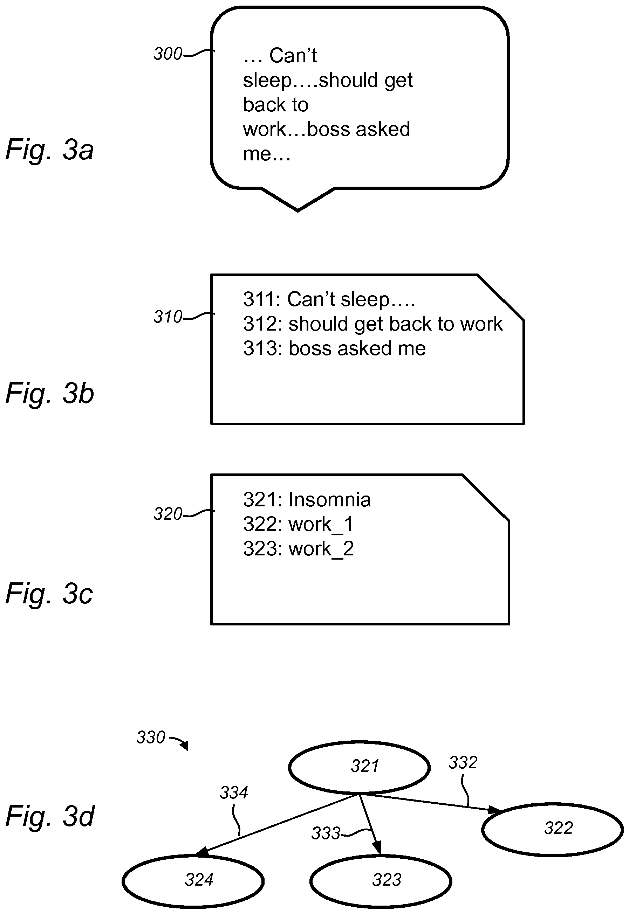

Figures 3a-3d illustrate an embodiment of system 200. **Figure 3a** schematically shows an example of an embodiment of a conversation 300. Only relevant parts of conversation 300 are shown. At the ellipses in conversation 300 there may be utterance that are unrelated to this example, or may be utterance of another speaker. In this case all speech shown in figure 3a is from the same speaker. **Figure 3b** schematically shows an example of an embodiment of speech recognition data 310. For example, speech recognition system such as unit 230 has transcribed the speech to text. In this example, the speech has also been portioned into individual utterances 311, 312 and 313. **Figure 3c** schematically shows an example of an embodiment of classified speech recognition data 320. Topics have been assigned to the utterances in speech data 310. For example, the utterance 'can't sleep' has been classified to the topic insomnia. The utterances 312 and 313 have each been classified with the topic work, but have also been assigned an identifier indicating a subdivision with the topic work. For example, the subdivision_1, in work_1, may mean work in general, whereas the subdivision_2, in work_2, may mean the relationship with the employer. **Figure 3d** schematically shows an example of an embodiment of a topic tree 330. Such a topic tree may be used to represent correlations. For example, topic 321 has a correlation with three other topics: topics 324, 323 and 322. The correlation between two topics may be expressed on an edge between them, e.g., correlations 334, 333, and 332.

For example, the relationship between insomnia and the two work related topics may be strong. Which may be shown by a high value for correlations 333 and 332. The correlation between topic 321 (insomnia) and topic 324 may be low (say, alcohol).

A clinician may use this information during anamnesis and/or to reach a better diagnosis. For example, when the user comes to see the doctor to talk about his insomnia, the doctor can consult this information, e.g., from database 250, e.g., through system 260, and find that the topic work is related to insomnia. The doctor can then ask questions about this topic. Accordingly, this tool can help overcome anxiety for the user to bring up this topic-the clinician is already aware that this may be a related topic; this topic can improve anamnesis if the patient did not associate work and insomnia and might not bring it up. On the other hand, other possible relationship may need to be explorer less deeply. For example, the tool does not need to press the doctor into detailing alcohol use, if there is no suspicion of its relevancy. This topic may be handled by the clinician more cursory than the work topic, for which relevancy is suggested by system 200. Note that as a result of an improved anamnesis it is not unlikely that another diagnosis comes out. If work related problems are at the cause of insomnia, clearly another intervention may be required than if poor sleep-hygiene is the culprit, e.g., alcohol use.

The clinician may also be provided with multiple second topics that can be related to a first topic, such as a first medical topic, e.g., insomnia. For example, in this case, the topics, work in general, and relationship with employer may both be presented. The order in which the topics are shown may be based on the strength of the correlation, but other orderings can be advantageous. For example, the second topics may be rated according to a rating criterion, and displayed in an order determined by the rating. The rating may be awkwardness. For example, a rating may estimate which topics a patient is more hesitant to discuss. This may help the clinician to approach the less-easily discussed topics later when the patient is more at ease. The system may monitor the comfort level of a patient, both during recording conversation and during a further conversation. Rating may be based on awkwardness, e.g., as determined by physiological measurements, or based or the order of mentioning, or known social acceptance as described. The rating criterion may be according to clinical relevance. For example, topics that have high diagnostic value may considered first.

Awkwardness can be associated to a topic by the system. Awkwardness may be pre-defined. For example, topics may have a predetermine awkwardness rating. For example, prescription drugs may have a lower predefine awkwardness than recreational drugs. However, in an embodiment, awkwardness is determined, e.g., estimated per
patient, e.g., to obtain a per-topic, per-patient awkwardness rating. Patient specific awkwardness may be determined from writing style, talking style and the frequency with which a topic is mentioned.

For example, the system, e.g., the topic classifier, can detect if the patient is shy or insecure when talking about certain symptoms, e.g., during or before the consult, by using various measurement technologies, e.g. facial expression recognition, voice and speech analysis, stress level, e.g., via GSR, and context of patient, e.g., age, gender, alone or with relative, etc.

Interestingly, system 200 may comprise a speech recognition unit 270. This may be a further unit, but could also be the same as unit 230. Speech recognition unit 270 recognizes the speech during anamnesis and identifies topics in it, e.g., using classifier 240. In this way, the clinician can be provided with related topics during the conversation. For example, if the patient brings up insomnia, the clinician can be warned, e.g., through display 214 that work is a related topic. In this way, the clinician can choose to bring up this topic, even if the patient does not.

Clinical system 260 may provide further diagnosis support. A medical decision tree may be stored, e.g., in an electronic storage, e.g., in system 260. The medical decision tree is arranged to diagnose a medical condition. Interestingly, some aspects of the tree can be highlighted based on the topics that have been detected. For example, a medical decision tree for insomnia may contain a question regarding stressors or regarding substance use. If work is correlated then the first may be highlighted, if alcohol is correlated than the latter may be highlighted.

For example, a part of the decision tree that is not relevant may not even be displayed. For example, a part of the insomnia decision tree regarding respiratory problems may be reduced or summarized. This gives more prominence to parts of the decision tree that are more likely. For example, the reduced or summarized or removed parts may be opened by the clinician if these prove relevant after all.

Based on found topics, system 200, e.g., system 260 may provide a possible diagnosis, and/or may provide a differential diagnosis. For example, based on the topics found, and/or the topics associated with medical topics a diagnosis for disease 1 may be conjectured. This possibility may be presented to the clinician, e.g., through system 260. For example, multiple diagnoses may be conjectured and shown to the clinician. In addition to a possible diagnosis, the system may present further symptoms that can confirm the diagnosis.

Figure 4a schematically shows an example of an embodiment of a medical decision tree 500. The tree may comprise multiple medical decision points, shown are medical decision point 511-519. There may be further decision points shown at 520. There may be more or fewer than the number of points shown in these figures.

For example, a decision point may be whether or not the patient has a fever, whether or not there is burning with urination, whether or not drugs were used (e.g., recreational or prescription), whether or not there is stress at work, etc. For example, a decision tree may be specific for a particular class of medical diagnosis, e.g., relating to insomnia, relating to STI or STD, and so on.

At the bottom of the tree there are possible medical diagnoses that are consistent with the medical decisions above it. For example, if stress at work and insomnia is found, then a possible diagnosis may be that this is work-related insomnia. These may also include a suggested course of action, e.g., counseling, cognitive therapy, and the like. For example, the medical decision tree may be arranged to diagnose a medical condition, and may be provided, e.g., stored, in an electronic storage. Medical decision tree 500 may be shown to the clinician, e.g., on a display. Medical decision tree reduces the chance that some causes may be neglected, e.g., forgotten, by the clinician, and improves that chance that care is given that conforms to agreed best-practices.

A conversation between the patient and the clinician, e.g., during anamnesis, may be recorded and analyzed for topics. For example, using a speech recognition algorithm. Accordingly, a medical topic may be identified, e.g., a first topic. This may be a topic that the patient and the clinician are currently conversing about. Alternatively, the clinician may provide the first topic, e.g., type in the first topic, or select it from a menu, etc., e.g., in system 260. Triggered by the first topic, one or more second topics may be identified, e.g., topics that have been correlated with this first topic for this particular patient, based on past recorded conversations.

Thus, if the first (or second topic) is mentioned during the conversation, this may trigger the mention of the second (or first) topic to the clinician. Note that, in an embodiment, not all topics are shown all time: not all possible topics, and not even all patient-specific topics. If a patient comes in for an injured knee, the system does not need to highlight, say an STD that the patient may also suffer from, as this disease is not the topic of the consult. However, once the patient mentions something related to this topics, e.g., if the first topic is triggered, then this may be shown, possibly along with correlated secondary topics.

Awkwardness can be associated to a topic. For example, the system, e.g., the topic classifier, can detect if the patient is shy or insecure when talking about certain symptoms, e.g., during or before the consult, by using various measurement technologies, e.g. facial expression recognition, voice and speech analysis, stress level, e.g., via GSR, and context of patient, e.g., age, gender, alone or with relative, etc.

When the second topic is found, it may be displayed to the clinician. Alternatively (or in addition), medical decision tree 500 may be adapted to take the second topic into account. For example, a part of medical decision tree 500 may be selected that comprises the first and the second topic. This part may be shown on a display to the clinician. For example, the part may be highlighted in the tree. For example, parts of the tree outside the selected part may not be shown or may be abbreviated. Figure 4b schematically shows an example of an embodiment of a medical decision tree 501. In tree 501, a part 540 of the tree is selected based on the first and second topic. For example, box 511, marked 'A' may correspond to the medical topic insomnia, while part 515 marked 'B' may correspond to work-stress. Accordingly, those parts of the tree that now seem less relevant are not selected. Selection 540 may be imparted to the clinician in a number of way, e.g., by highlighting. Figure 4c schematically shows an example of an embodiment of a medical decision tree 502. In tree 502, the unselected parts have been collapsed. Tree 502 shows collapsed sub-trees 523 and 524. For example, a clinician may open these sub-trees by clicking on collapsed sub-trees 523 and/or 524.

In an embodiment, the system 200 may be configured to offer a possible diagnosis to the clinician based on the first and/or second topic. For example, based a strong correlation between the first and second topic or topics, the system, e.g., system 260 may be able to apply a medical decision tree. For example, a strong correlation between insomnia and alcohol, e.g., above a threshold, may allow the system to offer the possible diagnosis 'alcohol-related insomnia'. The system may provide multiple possible diagnoses if more than one diagnosis is possible or plausible.

System 260 may comprise a device, which may have a user interface, which may include well-known elements such as one or more buttons, a keyboard, display, touch screen, etc. The recording system may also have a user interface. The user interface may be arranged for accommodating user interaction for performing a recording of a conversation, to look up topics for a patient, etc.

Storage may be implemented as an electronic memory, say a flash memory, or magnetic memory, say hard disk or the like, or optical memory, e.g., a DVD. Storage may comprise multiple discrete memories together making up storage. Storage may be implemented as cloud storage.

Typically, system 200 and its various devices, e.g., recording system 220, clinical system 260, etc., each comprise a microprocessor which executes appropriate software stored at the device; for example, that software may have been downloaded and/or stored in a corresponding memory, e.g., a volatile memory such as RAM or a non-volatile memory such as Flash. The devices may, in whole or in part, be implemented in programmable logic, e.g., as field-programmable gate array (FPGA). The devices maybe implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc.

In an embodiment, the system comprises one or more of a recoding circuit, a speech recognizing circuit, a topic classifying circuit, a storage circuit, a clinician circuit, etc. The circuits implement the corresponding units described herein. The circuits may be a processor circuit and storage circuit, the processor circuit executing instructions represented electronically in the storage circuits.

A processor circuit may be implemented in a distributed fashion, e.g., as multiple sub-processor circuits. A storage may be distributed over multiple distributed sub-storages. Part or all of the memory may be an electronic memory, magnetic memory, etc. For example, the storage may have volatile and a non-volatile part. Part of the storage may be read-only.

**Figure 2b** schematically shows an example of an embodiment of a device for medical diagnosis assistance 283. As pointed out above, there are many ways in which the components of system 200 can advantageously be distributed over devices. One particular advantageous way is shown in figure 2b.

Device 283 for medical diagnosis assistance shown in figure 2b has a recording interface 281 configured for receiving a conversation of a patient. For example, a user may install software 216 on a mobile computing device, e.g., a smart phone, or on some other device, e.g., a desktop, etc. A recording of a conversation may be transmitted to device 283. On the other hand, device 283 may also be part of the conversation, in this case device 283 can naturally record the conversation, as it is part of it. For example, device 283 may run a chatbot to which a user can connect through interface 281. For example, device 283 may support communication with a person, e.g., a medical professional.

Device 283 is similar to system 200. For example, device 283 may analyze the written or spoken conversation to identify topics, and to correlate topics. A database 250 may be used to store the found any one of conversations, identified topics and correlations.

Device 283 comprises a providing interface 282 configured for providing the first and/or second topic. For example, device 283 may transmit a report, e.g., comprising the first topic and one or more second topics that are correlated, e.g., to the clinician. For example, providing interface 282 may be configured to receive a request for the topics. For example, the request may comprise the first topic. In response to a request with the first topic the device 283 may respond with the second topic. For example, a clinician may send a request to device 283 from his clinical system 260. Clinical system 260 may comprise, e.g., a desktop or the like of the clinician. For example, interfaces 281 and 282 may be APIs.

Interestingly, device 283 may be configured for additional privacy protection. For example, database 250 may store, in addition to second topics found in recorded conversation of the patient, one or more default or dummy second topics. These default second topics may be predetermined, e.g., programmed in device 283. The default second topics may be selected from second topics of another user. For example, a default second topic may be obtained by selecting a random patient for whom the first topic is relevant and taking a correlated second topic therefrom. This provides the system with plausible deniability.

For example, if a clinician sends a request with first topic insomnia, then the device 283 may look up in database 250 that alcohol is valid second topic that is correlated for this user to the topic insomnia. However, in addition, or occasionally instead, device 283 may report the second topic work. Thus the clinician may receive the second topics: work and alcohol. The clinician knows that there is a chance that some of these are not valid for this user. A patient can thus plausibly deny a particular topic should he still desire to do so. These privacy protection may also be added to system 200. On the other hand, the clinician is forced to still go through an anamnesis, although he receives topics to discuss, he cannot fully rely only on the system, but is forced for make his own assessment.

Further embodiments, options and alternatives are described below.

In an embodiment, the system can detect if the patient is shy or insecure when talking about certain symptoms, including during or before the consult, by using measurement technologies, such as facial expression recognition, voice and speech analysis, stress level, e.g., via Galvanic Skin Response (GSR), and context of patient, e.g., age, gender, alone or with relative. Based on this awkwardness, the system can determine which topics are likely to be the ones non-disclosed during the consult, in response the system can, e.g., highlight them in the data tree.

The system may use speech/keyword recognition and text analysis to build a data tree specific to the patient around topics related to health or well-being. The tree structure for a particular topic, e.g., a disease, an anxiety, a wish for behavior change, may start from first mentioned items, e.g., more apparent symptom, or more socially accepted symptom, or more commonly known symptom, to last mentioned items, e.g., minor symptom, or shameful symptom, or symptom not commonly recognized. The first mentioned items can be identified as the ones disclosed most easily or more often by the patient. The following items can be classified based on the order in which the patient disclosed them and/or the number of overlap in associated searches, e.g., related themes, and/or the shame/stress related to the items, e.g., from text analysis or physiological parameters.

The system can retrieve a data tree for the general population. For example, such a tree may be constructed from searches, e.g., internet searches and classifying what people are looking for with respect to a given topic and importance of items. In this embodiment the system overlays the patient's data tree to the typical data tree related to a certain topic and identifies the items that are out of the scope, but yet related as they are shameful to talk about or vaguely related. The system may give a score to items based on their match with the items related to the topic, possibly annotated to show how sensitive this topic is.

A number of use cases are presented below.

For example, in a regular known type of anamnesis from a clinician, this could be the order of the patient's answers:
▪ 'I don't sleep well' > 'I wake up in the middle of the night' > 'I can't stop thinking about work' > 'I have too much work' > 'I feel pressured by my boss' > 'my boss threatens me'

For the clinician to arrive to the last answer of the patient, may require many questions, and also that the patient feels safe enough to open up. The root cause of the problem has to do with the work environment; missing this information could lead to treating only the symptoms, e.g., by prescribing sleeping pills, instead of treating the root cause, e.g., reaching to company mediator.

Using an embodiment, the system has heard and analyzed a similar discussion between the patient and a friend, where they discussed sleep and thoughts about work and boss. The system may have built a tree:
▪ Bad sleep > recurring thoughts about work > distressing boss

The system displays this tree to the clinician, who can ask directly after the first answer ('I don't sleep well'): 'is something worrying you, for instance at work? I know how stressful some situations can be.' Thus, anamnesis is quicker and more accurate.

In another example:
▪ 'I have belly pain' > 'It burns when I go to the toilet' > 'I think I might have a sexual disease' > related events...

The system may classify these symptoms as sensitive and knows that the patient might be reluctant to formulate other symptoms or the past related event. The system could present to the clinician a tree with the common diseases that are associated with the symptom that the patient mentioned, e.g., in past discussion or searches not necessarily to the clinician, in addition to the other symptoms for these possible diseases, so they can guide their anamnesis accordingly:
▪ Disease 1 > symptom patient + other possible symptom 1 + other possible symptom 2
▪ Disease 2 > symptom patient + other possible symptom 1 + other possible symptom 3
▪ Disease 3 > symptom patient + other possible symptom 2 + other possible symptom 4

In an embodiment, the system may determine a patient's medical literacy by examining the words they use in discussions or online searches, and displays these to the clinician for each step in the data tree so that the care professional can use appropriate terminology when talking to the patient.

In an embodiment,
- the patient reaches out to the GPs office, e.g., by phone or by chat. A virtual assistant is configured to start a conversation with the patient and asks the patient to describe the symptoms. It also has access to the patient's search history and past discussions for additional contextual information.
- Based on this, the system may propose a decision tree-type UI that allows the user to have a step-by-step guide to describe the symptom.
- If the system understands the diagnosis, or the patient has finished the decision tree, the system can formulate the description with proper medical terms, which may be disclosed to the patient.
- If the patient confirms, this description will be handed off by the virtual assistant to the doctor, so when the patient comes in the doctor's office for a face-to-face meeting, the doctor is already aware of the situation without the patient explaining it again

An embodiment allows a clinician to retrieve, from an initial patient complaint or comment during a consult, the underlying complaints or worries of the patient, based on previous conversations and/or online searches of the patient.

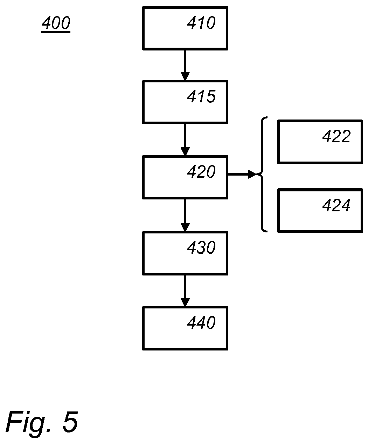

**Figure 5** schematically shows an example of an embodiment of a computer-implemented method 400 for medical diagnosis assistance. Method 400 for medical diagnosis assistance may be computer-implemented, e.g., in a system such as system 200. Method 400 comprises
- recording (410) a conversation of a patient using an electronic recording system,
- identifying (420) in the recorded conversation a plurality of topics, and classifying (422) a first topic in the plurality of topics as a medical symptom,
- determining (424) a second topic in the plurality of topics that is correlated with the first topic in the recorded conversation,
- providing (440) the first and second topic to a display to be presented to a clinician.

Method 400 may further comprise
- executing (415) a speech recognition algorithm configured to generate speech recognition data, the conversation being a spoken conversation, said plurality of topics being identified from the speech recognition data.
- storing (430) the first and/or second topic in a record of a patient database.

Method 400 may further comprise any of the operations disclosed herein.

Many different ways of executing the method are possible, as will be apparent to a person skilled in the art. For example, the steps can be performed in the shown order, but the order of the steps may also be varied or some steps may be executed in parallel. Moreover, in between steps other method steps may be inserted. The inserted steps may represent refinements of the method such as described herein, or may be unrelated to the method.

Embodiments of the method may be executed using software, which comprises instructions for causing a processor system to perform method 400. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the invention also extends to computer programs, particularly computer programs on or in a carrier, adapted for putting the invention into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the means of at least one of the systems and/or products set forth.

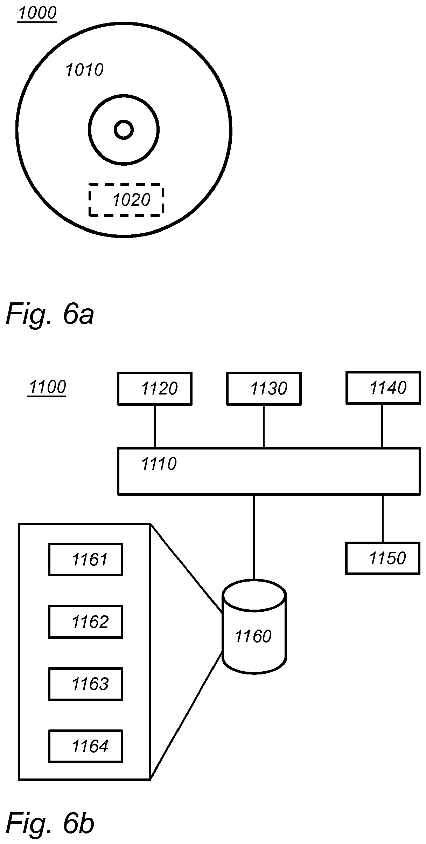

**Figure 6a** shows a computer readable medium 1000 having a writable part 1010 comprising a computer program 1020, the computer program 1020 comprising instructions for causing a processor system to perform a method for medical diagnosis assistance, according to an embodiment. The computer program 1020 may be embodied on the computer readable medium 1000 as physical marks or by means of magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said method for medical diagnosis assistance.

**Figure 6b** illustrates an exemplary hardware diagram 1100 for implementing a device according to an embodiment. As shown, the device 1100 includes a processor 1120, memory 1130, user interface 1140, communication interface 1150, and storage 1160 interconnected via one or more system buses 1110. It will be understood that this figure constitutes, in some respects, an abstraction and that the actual organization of the components of the device 1100 may be more complex than illustrated.

The processor 1120 may be any hardware device capable of executing instructions stored in memory 1130 or storage 1160 or otherwise processing data. As such, the processor may include a microprocessor, field programmable gate array (FPGA), application-specific integrated circuit (ASIC), or other similar devices. For example, the processor may be an Intel Core i7 processor, ARM Cortex-R8, etc. In an embodiment, the processor may be ARM Cortex M0.

The memory 1130 may include various memories such as, for example L1, L2, or L3 cache or system memory. As such, the memory 1130 may include static random access memory (SRAM), dynamic RAM (DRAM), flash memory, read only memory (ROM), or other similar memory devices. It will be apparent that, in embodiments where the processor includes one or more ASICs (or other processing devices) that implement one or more of the functions described herein in hardware, the software described as corresponding to such functionality in other embodiments may be omitted.

The user interface 1140 may include one or more devices for enabling communication with a user such as an administrator. For example, the user interface 1140 may include a display, a mouse, and a keyboard for receiving user commands. In some embodiments, the user interface 1140 may include a command line interface or graphical user interface that may be presented to a remote terminal via the communication interface 1150.

The communication interface 1150 may include one or more devices for enabling communication with other hardware devices. For example, the communication interface 1150 may include a network interface card (NIC) configured to communicate according to the Ethernet protocol. For example, the communication interface 1150 may comprise an antenna, connectors or both, and the like. Additionally, the communication interface 1150 may implement a TCP/IP stack for communication according to the TCP/IP protocols. Various alternative or additional hardware or configurations for the communication interface 1150 will be apparent.

The storage 1160 may include one or more machine-readable storage media such as read-only memory (ROM), random-access memory (RAM), magnetic disk storage media, optical storage media, flash-memory devices, or similar storage media. In various embodiments, the storage 1160 may store instructions for execution by the processor 1120 or data upon with the processor 1120 may operate. For example, the storage 1160 may store a base operating system 1161 for controlling various basic operations of the hardware 1100. For example, the storage may store instructions 1162 for recording a conversation, instructions 1163 to identify in the recorded conversation a plurality of topics, and/or instructions 1164 to and determining first and a second topic in the plurality of topics that are correlated, and so on, etc.

It will be apparent that various information described as stored in the storage 1160 may be additionally or alternatively stored in the memory 1130. In this respect, the memory 1130 may also be considered to constitute a "storage device" and the storage 1160 may be considered a "memory." Various other arrangements will be apparent. Further, the memory 1130 and storage 1160 may both be considered to be "non-transitory machine-readable media." As used herein, the term "non-transitory" will be understood to exclude transitory signals but to include all forms of storage, including both volatile and non-volatile memories.

While device 1100 is shown as including one of each described component, the various components may be duplicated in various embodiments. For example, the processor 1120 may include multiple microprocessors that are configured to independently execute the methods described herein or are configured to perform steps or subroutines of the methods described herein such that the multiple processors cooperate to achieve the functionality described herein. Further, where the device 1100 is implemented in a cloud computing system, the various hardware components may belong to separate physical systems. For example, the processor 1120 may include a first processor in a first server and a second processor in a second server.

It should be noted that the above-mentioned embodiments illustrate rather than limit the invention, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

In the claims, references in parentheses refer to reference signs in drawings of exemplifying embodiments or to formulas of embodiments, thus increasing the intelligibility of the claim. These references shall not be construed as limiting the claim.

## Claims

1. A computer-implemented method (400) for medical diagnosis assistance, the method comprising
- recording (410) one or more conversations of a patient using an electronic recording system,
- identifying (420) multiple topics in the one or more recorded conversations thus obtaining a plurality of patient-specific topics,
- classifying (422) a first topic in the plurality of patient-specific topics as a medical symptom,
- determining (424) a second topic in the plurality of patient-specific topics that is correlated with the first topic in the one or more recorded conversations of the patient,
- in response to a trigger for the first topic, providing (440) information associated with the second topic to a display to be presented to a clinician.

2. A method as in claim 1, comprising
- recording a further conversation between the patient and the clinician, the further conversation being a spoken conversation,
- applying a speech recognition algorithm to the further recorded conversation of the patient for generating speech recognition data,
- identifying the first topic in the speech recognition data to trigger providing the information associated with the correlated second topic.

3. A method as in any one of the preceding claims comprising
- providing a medical decision tree arranged to diagnose a medical condition in an electronic storage,
- selecting a part of the medical decision tree comprising the first and the second topic, and providing the part to the display.

4. A method as in any one of the preceding claims, wherein the recording system comprises a software application installed on a mobile computing device and/or a smart speaker.

5. A method as in any one of the preceding claims, comprising
- applying (415) a speech recognition algorithm to a conversation of the one or more recorded conversations, the conversation being a spoken conversation, said speech recognition algorithm being configured to generate speech recognition data, topics for the plurality of patient-specific topics being identified from the speech recognition data.

6. A method as in any one of the preceding claims, wherein a conversation of the one or more recorded conversations is a written conversation, topics for the plurality of patient-specific topics being identified from the written conversation.

7. A method as in any one of the preceding claims, wherein a conversation of the one or more recorded conversations is between a patient and a chatbot.

8. A method as in any one of the preceding claims, comprising
- recording one or more searches of the patient in an electronic information system, and identifying in the recorded one or more searches further topics for the plurality of patient-specific topics.

9. A method as in any one of the preceding claims comprising
- storing (430) the first and/or second topic in a record of a patient database.

10. A method as in any one of the preceding claims comprising
- determining a plurality of second topics in the plurality of patient-specific topics that are correlated with the first topic in one or more recorded conversations,
- rating the plurality of second topics according to a rating criterion,
- providing at least part of the plurality of second topics to the display in an order determined by the rating.

11. A method as in any one of the preceding claims, comprising
- determining multiple medical conditions consistent with the first and/or second topic,
- determine a further symptom of the multiple medical conditions,
- provide the multiple medical conditions and the further symptom to the display.

12. A transitory or non-transitory computer readable medium (1000) comprising data (1020) representing instructions to cause a processor system to perform the method according to any one of the preceding claims.

13. An electronic system (200) for medical diagnosis assistance, the system comprising
- a recording unit (220) configured for recording one or more conversations of a patient,
- a processor system configured for
- identifying multiple topics in the one or more recorded conversations thus obtaining a plurality of patient-specific topics,
- classifying a first topic in the plurality of patient-specific topics as a medical symptom,
- determining a second topic in the plurality of patient-specific topics that is correlated with the first topic in the one or more recorded conversations of the patient,
- in response to a trigger for the first topic, providing information associated with the second topic to a display to be presented to a clinician.

14. An electronic device (283) for medical diagnosis assistance, the device comprising
- a recording interface (281) configured for receiving one or more conversations of a patient,
- a processor system configured for
- identifying multiple topics in the one or more recorded conversations thus obtaining a plurality of patient-specific topics,
- classifying a first topic in the plurality of patient-specific topics as a medical symptom,
- determining a second topic in the plurality of patient-specific topics that is correlated with the first topic in the one or more recorded conversations of the patient, and
- a providing interface (281) configured for, in response to a trigger for the first topic, providing (440) information associated with second topic.
